Europäisches Patentamt

European Patent Office

Office européen des brevets

(11) Veröffentlichungsnummer: **0 076 895**
A2

(12) **EUROPÄISCHE PATENTANMELDUNG**

(21) Anmeldenummer: 82106705.5

(22) Anmeldetag: 24.07.82

(51) Int. Cl.³: **B 65 D 25/10**, A 61 L 2/26

(30) Priorität: 06.10.81 CH 6408/81

(71) Anmelder: **GEBRÜDER SULZER AKTIENGESELLSCHAFT**, Zürcherstrasse 9, CH-8401 Winterthur (CH)

(43) Veröffentlichungstag der Anmeldung: 20.04.83 Patentblatt 83/16

(72) Erfinder: **Frey, Otto, Walrütistrasse 56, CH-8400 Winterthur (CH)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf (DE)**

(84) Benannte Vertragsstaaten: **AT DE FR GB IT NL**

(54) **Behälter für die Aufbewahrung und den Transport von sterilen Implantat-Teilen.**

(57) In das Gefäß (1) des Behälters ist ein Klemmdeckel (7) einsetzbar, der mit Hilfe einer Verzahnung (9) fixiert wird, und das Implantat (2) gegen den Boden (6) des Gefäßes (1) preßt. Die innere Fläche des Klemmdeckels (7) ist als Lagerschale (13) für das Implantat (2) ausgebildet.

Durch die Fixierung des Implantates (2) im Gefäß (1) werden Beschädigungen der Schweißnaht der Verschlußfolie (3) des Gefäßes (1) vermieden; weiterhin wird die Präsentation des Implantates (2) während der Operation erleichtert. Beide Wirkungen haben den Zweck, die Gefahr eines Sterilitätsverlustes für das Implantat (2) zu minimalisieren.

EP 0 076 895 A2

P. 5662/Wg/IS

Gebrüder Sulzer, Aktiengesellschaft, Winterthur/Schweiz

Behälter für die Aufbewahrung und den Transport von
sterilen Implantat-Teilen

Die Erfindung betrifft einen Behälter für die Aufbewahrung und den Transport von sterilen Implantat-Teilen, insbesondere von künstlichen Hüftgelenkspfannen, bestehend aus einem topfartigen Gefäss, das an einer Seite durch eine abreissbare Folie verschlossen ist, die mit dem Rand des Behälters verklebt oder verschweisst ist.

An die Gasdichtheit von Verpackungen und Behältern für sterile Implantat-Teile werden sehr hohe Anforderungen gestellt, um die Gefahr einer Verunreinigung durch mit eindringendem Gas transportierte Mikroben auszuschliessen oder zumindest auf ein Minimum zu beschränken. Implantat-Teile, wie z.B. Hüftgelenkspfannen, werden unter anderem in Behältern der vorstehend genannten Art verpackt und in verpackter Form durch Gamma-Strahlung sterilisiert, wobei der geschilderte Behälter sehr häufig noch von weiteren Schutzverpackungen umschlossen ist. Das Implantat ist in einem solchen Behälter bisher frei beweglich; durch Stösse während des Transports gegen die, im allgemeinen aufgeschweisste Folie besteht die Gefahr geringfügiger Beschädigungen der Schweissnaht, die die Sterilität des Implantats beeinträchtigen können.

Weiterhin werden die genannten Behälter während der Operation im allgemeinen von unsterilen Personen geöffnet und einer sterilen Person übergeben, die das sterile Implantat aus dem Behälter entnimmt. Da das Implantat bei bisherigen Behältern relativ unzugänglich gelagert ist, besteht auch bei dieser Entnahme die Gefahr, dass die Sterilität beeinträchtigt wird.

Aufgabe der Erfindung ist es daher, einen Behälter zu schaffen, in dem das Implantat auf einfache Weise fixiert und nach dem Oeffnen des Behälters leicht zugänglich dargeboten werden kann. Mit der vorliegenden Erfindung wird diese Aufgabe gelöst durch einen im Innenraum des Gefässes fixierbaren Klemmdeckel zum Anpressen des Implantates am Boden des Gefässes und durch eine Formgebung der dem Implantat zugewandten Klemmdeckelfläche derart, dass nach einer Drehung des Gefässes um 180° das Implantat auf dem Klemmdeckel gelagert ist.

Durch das Anpressen am Boden des Gefässes ist das Implantat mit Hilfe des Klemmdeckels während des Transports fixiert; darüberhinaus ist der Klemmdeckel eine "Zwischenwand", so dass das Implantat überhaupt nicht mehr direkt mit der aufgeschweissten Folie in Berührung kommen kann. Der Klemmdeckel bildet weiterhin nach einer Drehung des Gefässes um 180° eine tablettartige Lagerfläche, auf der das Implantat von der unsterilen Person während der Operation der sterilen Person dargeboten werden kann; diese kann das Implantat dann von dem "Tablett" ohne Schwierigkeiten einfach abheben, wobei die Möglichkeit, dass es zu Streifberührungen mit der unsterilen Person kommt, praktisch ausgeschlossen ist.

Eine vorteilhafte Ausführungsform für die Befestigung des Klemmdeckels im Gefäss ergibt sich, wenn der Innenmantel des Gefässes eine Verriegelungs-Verzahnung hat, die in Umfangsrichtung durch mindestens zwei Führungsnuten unterbrochen ist, und wenn der Aussenmantel des Klemmdeckels an die Führungsnuten in Form und Abmessungen angepasste Umfangsabschnittebesitzt, die mit einer entsprechenden Gegenverzahnung versehen sind, während die Oberfläche des Klemmdeckels als Lagerfläche einfach eine muldenartige Vertiefung haben kann.

Unter Umständen ist es - zu besseren Fixierung des Implantates im Behälter - zweckmässig, wenn der Boden des Gefässes ein Profil aufweist, das Horizontalbewegungen des Implantates zumindest begrenzt. Das Verriegeln und Lösen des Klemmdeckels lässt sich erleichtern, wenn der Klemmdeckel auf der Aussenfläche einen Handgriff hat.

Im folgenden wird die Erfindung anhand eines Ausführungsbeispiels im Zusammenhang mit der Zeichnung näher erläutert.

        Fig. 1 zeigt einen Behälter im Längsschnitt entlang der Schnittlinie I-I von Fig. 2;

        Fig. 2 ist eine Aufsicht auf den Behälter der Fig. 1 von oben, wobei die aufgeschweisste Verschlussfolie bereits entfernt ist;

        Fig. 3 schliesslich gibt eine Ansicht des Klemmdeckels auf seine Innenseite wieder.

Das Gefäss 1, das eine Hüftgelenkspfanne 2 enthält, ist auf seiner Oberseite mit einer Folie 3 abgedeckt; diese ist mit dem Rand 4 des Gefässes 1 verschweisst oder verklebt und hat an einer Stelle eine Lasche 5 zum Aufreissen.

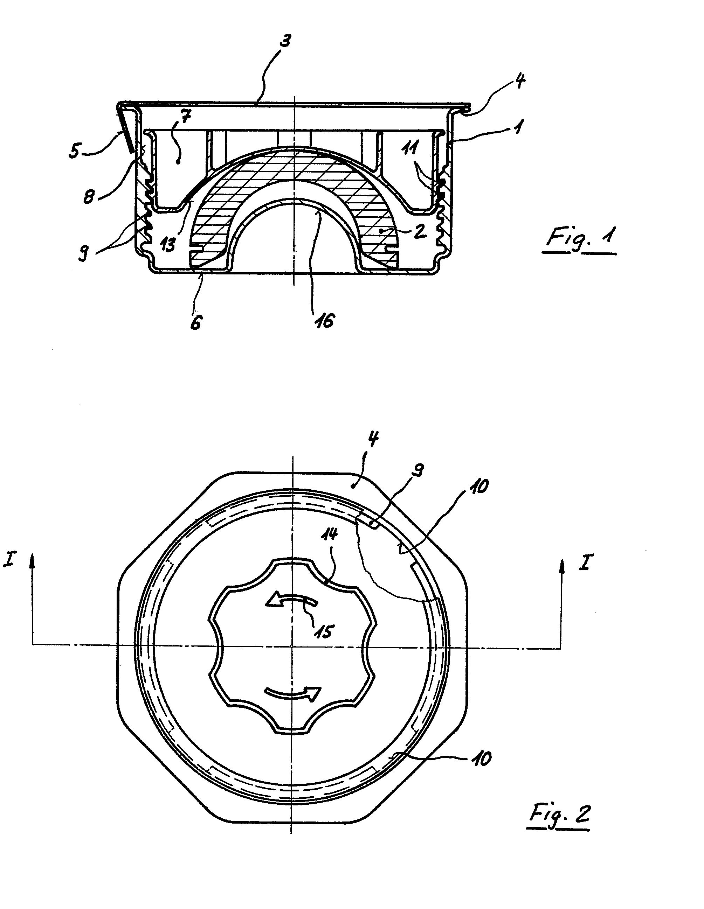

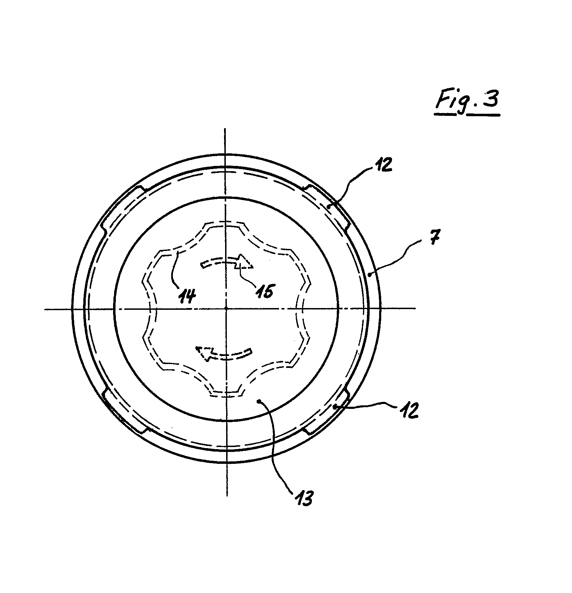

Gegen den Boden 6 des Gefässes 1 wird die Pfanne 2 mit Hilfe eines Klemmdeckels 7 gepresst; zu diesem Zweck ist der Klemmdeckel 7 in eine Verriegelungs-Verzahnung 9 eingeschraubt, die auf dem Umfang des Innenmantels 8
des Gefässes 1 gegen die Horizontale geneigt verläuft.
Für das Einsetzen des Deckels 7 ist die Verriegelungs-
Verzahnung 9 an vier um je 90$^O$ versetzten Stellen von
Führungsnuten 10 (Fig. 2) unterbrochen. In diese gleiten
beim Schliessen des Klemmdeckels die eine Gegenverzahnung
11 tragenden Abschnitte 12 (Fig. 3) des Aussenumfangs des
Deckels 7. Die Abschnitte 12 wie die Gegenverzahnung 11
sind in Form und Winkellage zueinander an die Lage der
Führungsnuten im Gefäss 1 angepasst; es ist jedoch nicht
zwingend, dass Abschnitte 12 und Führungsnuten 10 an Zahl
übereinstimmen.

Die dem Implantat 2 zugewandte Fläche des Klemmdeckels 7
ist als flache schalenartige Mulde 13 ausgebildet, in
der, wie beschrieben, das Implantat 2 nach Drehen des
Gefässes um 180$^O$ relativ stabil gelagert während der
Operation angereicht werden kann.

Auf seiner Aussenfläche hat der Klemmdeckel 7 einen zentralen Handgriff 14 (Fig. 2) sowie Pfeile 15, durch die
die Drehrichtung für ein Lösen des Klemmdeckels angezeigt
ist.

Der Boden des Gefässes hat ein zentrales halbkugelförmiges
Profil 16, das in die Gelenkkopfschale der Pfanne 2 eingreift und die Möglichkeiten der Hüftgelenkpfanne 2 für
Horizontalbewegungen eng begrenzt. Der zwischen dem Innenmantel 8 des Gefässes 1 und den Profil 16 sowie zwischen
dem Profil und dem Klemmdeckel verbleibende Hohlraum sind
darüberhinaus so bemessen, dass Implantate 2 unterschiedlicher Grössen im gleichen Gefäss 1 untergebracht und
transportiert werden können.

Als Material für den Behälter hat sich gegen Strahlungssterilisation beständiger Kunststoff, wie z.B. Styrol-
Butadien Copolymere, bewährt, der beispielsweise nach
einem bekannten Spritzgussverfahren verarbeitet wird.

Patentansprüche

1. Behälter für die Aufbewahrung und den Transport von sterilen Implantat-Teilen, insbesondere von künstlichen Hüftgelenkspfannen, bestehend aus einem topfartigen Gefäss, das an einer Seite durch eine abreissbare Folie verschlossen ist, die mit dem Rand des Behälters verklebt oder verschweisst ist, gekennzeichnet durch einen im Innenraum des Gefässes (1) fixierbaren Klemmdeckel (7) zum Anpressen des Implantates (2) am Boden (6) des Gefässes (1) und durch eine Formgebung der dem Implantat (2) zugewandten Klemmdeckelfläche derart, dass nach einer Drehung des Gefässes (1) um $180^{\circ}$ das Implantat (2) auf dem Klemmdeckel (7) gelagert ist.

2. Behälter nach Anspruch 1, dadurch gekennzeichnet, dass der Innenmantel (8) des Gefässes (1) eine Verriegelungs-Verzahnung (9) hat, die in Umfangsrichtung durch mindestens zwei Führungsnuten (10) unterbrochen ist, und dass der Aussenmantel des Klemmdeckels (7) an die Führungsnuten (10) in Form und Abmessungen angepasste Umfangsabschnitte (12) besitzt, die mit einer entsprechenden Gegenverzahnung (11) versehen sind.

3. Behälter nach Anspruch 1 oder 2, dadurch gekennzeichnet, dass die dem Implantat (2) zugewandte Klemmdeckelfläche als Lagerfläche für das Implantat (2) eine muldenartige Vertiefung (13) hat.

4. Behälter nach einem der Ansprüche 1 - 3, dadurch gekennzeichnet, dass der Klemmdeckel (7) auf der Aussenfläche einen Handgriff (14) hat.

5. Behälter nach einem der Ansprüche 1 - 4, dadurch ge-

0076895

kennzeichnet, dass der Boden (6) des Gefässes (1) ein
Profil (16) aufweist, das Horizontalbewegungen des
Implantats (2) zumindest begrenzt.

*Fig. 1*

*Fig. 2*

0076895

*Fig.3*